Europäisches Patentamt

European Patent Office

Office européen des brevets

(11) Veröffentlichungsnummer: **0 190 176**
**B1**

(12) # EUROPÄISCHE PATENTSCHRIFT

(45) Veröffentlichungstag der Patentschrift: 22.11.90

(51) Int. Cl.⁵: **A 61 K 31/335**

(21) Anmeldenummer: 85903245.0

(22) Anmeldetag: **14.06.85**

(86) Internationale Anmeldenummer:
**PCT/EP85/00285**

(87) Internationale Veröffentlichungsnummer:
**WO 86/00805 13.02.86 Gazette 86/04**

(54) **Verwendung von Doxaminol zur Herstellung eines Arzneimittels zur Behandlung der peripheren arteriellen Verschlusskrankheit.**

(30) Priorität: 24.07.84 DE 3427209

(43) Veröffentlichungstag der Anmeldung:
**13.08.86 Patentblatt 86/33**

(45) Bekanntmachung des Hinweises auf die
Patenterteilung:
**22.11.90 Patentblatt 90/47**

(84) Benannte Vertragsstaaten:
**AT BE CH DE FR GB IT LI LU NL SE**

(56) Entgegenhaltungen:
FR-A-2 277 589

Chemical Abstracts, Band 100, Nr. 5, 30. Januar
1984, Columbus, Ohio, USA; M.A. Boogaerts et
al.: "Effect of beta-blocking drugs on red cell
adhesive and rheological properties", siehe
Seite 28, Zusammenfassung Nr. 29475c

Die Akte enthält technische Angaben, die nach
dem Eingang der Anmeldung eingereicht
wurden und die nicht in dieser Patentschrift
enthalten sind.

(73) Patentinhaber: BOEHRINGER MANNHEIM
GMBH
Sandhofer Strasse 116
D-6800 Mannheim 31 (DE)

(72) Erfinder: STREIN, Klaus
Eichenstrasse 15
D-6944 Hemsbach (DE)
Erfinder: MÜLLER-BECKMANN, Bernd
Hochgewanne 46
D-6718 Grünstadt (DE)
Erfinder: BÖHM, Erwin
In der Schanz 37
D-6905 Schriesheim (DE)

Courier Press, Leamington Spa, England.

**Beschreibung**

Doxaminol, dessen chemische Bezeichnung N-Methyl-N-[2-(6,11)-dihydrodibenz[b,e]oxepin-11-yl)ethyl]-1-amino-3-phenoxy-propanol-(2) lautet, ist eine bekannte Substanz, die auf die β-Rezeptoren wirkt und das Herzzeitvolumen erhöht. Sie ist Gegenstand der DE—PS 23 35 943. Strukturanaloge Verbindungen mit den gleichen Eigenschaften sind in der DE—PS 1 568 145 beschrieben, wobei auch aufgeführt ist, daß die angegebene Wirkung durch eine Entleerung venöser Blutspeicher zustande kommt.

Dies ist eine Wirkung, die für die Behandlung der Hypotonie (niedriger Blutdruck) und orthostatischer Beschwerden (insbesondere Blutdruckabfall bei Übergang aus der horizontalen in die vertikale Position (genutzt werden kann.

Es wurde nun überraschenderweise gefunden, daß Doxaminol die Rheologie (Fließeigenschaften) des Blutes beeinflußt. Dies hat zur Folge, daß zu der Behandlung der oben genannten Indikation der orthostatischen Beschwerden bzw. Hypotonie die Indikation periphere arterielle Verschlußkrankheit kommt.

Versuchsbericht

Bei einer Reihe von Erkrankungen, insbesondere bei peripheren und cerebralen Durchblutungsstörungen, sind die Fließeigenschaften des Blutes verschlechtert. Dies gilt vor allem für die Verformbarkeit der Erythrozyten. Da für die Passage der Erythrozyten durch das Kapillarsystem eine gute Verformbarkeit Voraussetzung ist, kommt es bei Durchblutungsstörungen aufgrund der verschlechterten Verformbarkeit und des zudem wegen der Durchblutungsstörungen verminderten Perfusionsdrucks zu einer starken Herabsetzung der Sauerstoffversorgung des Gewebes. Es ist daher ein wichtiges Ziel der Behandlung solcher Erkrankungen, Substanzen zu suchen, die die Verformbarkeit der Erythrozyten verbessern.

Die Verformbarkeit der Erythrozyten wird heute üblicherweise in sogenannten Filtrationstests gemessen. Dabei wird eine Suspension von Erythrozyten durch ein Filter geschickt, das Poren besitzt, die einen Durchmesser haben, der dem kleiner Kapillaren entspricht. Eine Substanz, die die Verformbarkeit der Erythrozyten verbessert, muß auch gleichzeitig die Passage der Erythrozyten durch diese Filterporen und damit die Filtrierbarkeit der Erythrozytensuspension erhöhen.

In einem Versuchsmodell wurde nun untersucht, inwieweit Doxaminol und auch WW 241, die aus der DE—PS 15 68 145 bekannte N-Methyl-freie Doxaminol-Verbindung, in Suspensionen von Humanerythrozyten die Verformbarkeit verändern. Die Humanerythrozyten stammen von Patienten mit peripheren und cerebralen Durchblutungsstörungen.

Methode

Patienten einer Angiologischen Klinik mit gesicherter peripherer bzw. cerebraler Durchblutungsstörungen wurde im Rahmen der ohnehin für die Routineuntersuchung durchzuführenden Blutabnahme zusätzlich 20 ml Blut/Patient für die Untersuchung auf Erythrozytenverformbarkeit abgenommen. Durch Vorlage von Heparin wurde eine Gerinnung des Blutes verhindert. Spätestens 2 h nach der Abnahme wurde das Blut zentrifugiert, die Erythrozyten wurden mit einer isotonen Pufferlösung zweimal gewaschen und dann in dieser Pufferlösung suspendiert. Dabei wurde ein Hämatokrit von 3% eingestellt. Diese Suspension wurde in mehreren Proben aufgeteilt, diese Proben wurden dann entweder eine der beiden oben genannten Substanzen zugefügt oder in der Kontrolle nur das Lösungsmittel für diese Substanzen. Nach einer 30minütigen Inkubation bei 37°C wurden die Proben in einer speziellen Einheit filtriert. Die Filtration erfolgte durch sogenannte Nukleoporefilter, deren Porenweite einheitlich 5 µm beträgt. Gemessen wurde die durch das Filter hindurchgelaufene Suspensionsmenge als Funktion der Zeit. Aus diesen Daten wird eine sogenannte Flußkonstante berechnet. Diese Flußkonstante gibt im Prinzip die Geschwindigkeit des Erythrozytenflusses durch das Nukleoporefilter während der ersten Phase der Filtration an. Sind die Erythrozyten schlechter verformbar, so hat diese Flußkonstante einen kleineren Zahlenwert. Im Extremfall wird diese Flußkonstante 0, dies erhalten wir z.B. dann, wenn die Verformbarkeit der Erythrozyten total aufgehoben wird, etwa durch Einwirken hoher Konzentrationen von Milchsäure.

Die Untersuchungen wurden an 10 Blutproben von 10 verschiedenen Patienten durchgeführt. Doxaminol und WW 241 wurden in den Konzentrationen $10^{-7}$ und $10^{-6}$ mol/l getestet. Diese Konzentrationen entsprechen in etwa den Konzentrationen, die im Blut nach therapeutischen Dosen von diesen beiden Substanzen zu erwarten sind.

EP 0 190 176 B1

## TABELLE 1

| Kontrolle Patientenblut | Doxaminol $10^{-7}$ mol/l | Doxaminol $10^{-6}$ mol/l | WW 241 $10^{-7}$ mol/l | WW 241 $10^{-6}$ mol/l | Kontrolle Blut von gesunden |
|---|---|---|---|---|---|
| $16,5 \pm 4,1$ | $19,2 \pm 3,7$ * | $20,0 \pm 2,8$ * | $16,3 \pm 5,0$ | $17,1 \pm 4,3$ | $21,2 \pm 4,1$ * |

Einfluß von Doxaminol und WW 241 auf die *Flußkonstante* von Erythrozytensuspensionen
Angegeben sind Mittelwerte ± Standardabweichung aus n = 10 Versuchen
WW 241 = N-[2-(6,11)-dihydrodibenz[b,e]oxepin-11-yl)ethyl]-1-amino-3-phenoxy-propanol-(2)

* bedeutet signifikanter Unterschied (p < 0,05) gegenüber der Kontrolle Patientenblut

Ergebnisse:
Die Ergebnisse sind in der Tabelle 1 zusammengestellt. Man erkennt, daß die Flußkonstante sowohl durch $10^{-6}$ als auch $10^{-7}$ mol/l Doxaminol erniedrigt wurde. Test auf Signifikans mit Hilfe des Wilcoxon-Paartestes ergab, daß die Effekte statistisch signifikant sind. Dagegen führte WW 241 zu keiner statistisch signifikanten Beeinflussung der Flußkonstanten. Interessant ist auch ein Vergleich der für Patientenblut erhaltenen Flußkonstanten mit der Flußkonstanten, die wir in einer Versuchsreihe erhielten, die mit Blut von gesunden freiwilligen Spendern durchgeführt wurde. Die Flußkonstante ist bei Verwendung von Patientenblut signifikant niedriger als bei Verwendung von Probandenblut. Dies bestätigt einmal mehr die bereits in der Literatur zitierten Befunde, wonach Patientenblut verringerte Erythrozytenverformbarkeiten hat. Doxaminol verändert die Filtrierbarkeit des Patientenblutes so, daß dieses sich annähernd so verhält, wie das Blut gesunder Pesonen.
In einer anderen Untersuchung, die an Patienten durchgeführt wurde, wurde Doxaminol mit einem Placebo verglichen, um den Einfluß auf die Fließeigenschaften des Blutes zu beobachten.

Versuchsbeschreibung
10 Patienten mti Blut, dessen Viskosität erhöht war, und einer verminderten Filtrierbarkeit des Blutes, wurden ausgewählt. 50 mg Doxaminol sowie Placebo wurden per os den 10 Patienten in einem Doppelblindversuch appliziert. Die Patienten nahmen das Arzneimittel bzw. das Placebo am Morgen. Blutproben wurden 30, 60 und 90 Minuten nach Einnahme der Mittel aus den Armvenen entnommen.
Die Blutproben wurden mit $K^+$-EDTA 10% (2 mg/ml Blut) ungerinnbar gemacht und die Gesamtblutviskosität und Filtrierbarkeit gemessen.
Die Gesamtblutviskosität wurde mit einem Kegel-Platte- 1/4 LVT Microviscometer (Well Brookfield) bei 450, 225, 90 und 45 $sec^{-1}$ Schergrad bei 37°C bestimmt.
Die Filtrierbarkeit des Blutes wurde mittels einer Filtrationsmethode durch Nucleopore-Membrane (13 mm) mit 5 µm Poren bei einem Unterdruck von 20 cm $H_2O$ und 37°C bestimmt.

Ergebnisse:
Die Ergebnisse nach Placebo-Gabe sind in Tabelle 2, die nach Gabe von Doxaminol in Tabelle 3 aufgeführt.
60 Minuten nach Einnahme von 50 mg Doxaminol wurde eine signifikante Verbesserung der Filtrierbarkeit des Blutes erreicht. Nach 90 Minuten war der Effekt noch weiter ausgeprägt.
Zu den gleichen Zeitpunkten wurde eine signifikante Abnahme der Blutviskosität beobachtet, die bei niedrigstem Schergrad (45 $sec^{-1}$) ermittelt wurde.
Klinische Studien mit 100 mg Doxaminol zeigten einen noch stärkeren Effekt als 50 mg.

# EP 0 190 176 B1

### TABELLE 2

| | | Ausgangswert | 30′ | ′t′ | 60′ | ′t′ | 90′ | ′t′ |
|---|---|---|---|---|---|---|---|---|
| Filtrierbarkeit ($V_{BC}$) | | $0.328 \pm 0.009$ | $0.328 \pm 0.009$ | 1.38 | $0.328 \pm 0.009$ | 2.04 | $0.328 \pm 0.009$ | 2.13 |
| Hämatokrit (%) | | $39.6 \pm 1.19$ | $39.5 \pm 1.18$ | 1.00 | $39.5 \pm 1.18$ | 1.5 | $39.5 \pm 1.13$ | 1.15 |
| Viskosität | $450s^{-1}$ | $3.79 \pm 0.06$ | $3.79 \pm 0.07$ | 0.25 | $3.77 \pm 0.06$ | 1.28 | $3.79 \pm 0.07$ | 0.23 |
| | 250 | $3.98 \pm 0.07$ | $3.99 \pm 0.08$ | 0.49 | $3.97 \pm 0.08$ | 0.72 | $3.99 \pm 0.07$ | 0.22 |
| (cPs) | 90 | $4.38 \pm 0.09$ | $4.38 \pm 0.10$ | 0.04 | $4.35 \pm 0.12$ | 0.75 | $4.33 \pm 0.11$ | 1.42 |
| | 45 | $4.78 \pm 0.12$ | $4.80 \pm 0.14$ | 0.66 | $4.77 \pm 0.13$ | 0.16 | $4.75 \pm 0.12$ | 0.68 |

t: Prüfgröße des t-Testes für gepaarte Daten (Test auf statische Signifikanz)

### TABELLE 3

| | | Ausgangswert | 30′ | ′t′ | 60′ | ′t′ | 90′ | ′t′ |
|---|---|---|---|---|---|---|---|---|
| Filtrierbarkeit ($V_{BC}$) | | $0.337 \pm 0.08$ | $0.356 \pm 0.01$ | 2.16 | $0.360 \pm 0.01$ | 2.34 * | $0.368 \pm 0.01$ | 3.84 ** |
| Hämatokrit (%) | | $39.7 \pm 1.20$ | $39.3 \pm 1.35$ | 0.87 | $39.2 \pm 1.39$ | 1.16 | $39.3 \pm 1.14$ | 0.96 |
| Viskosität | $450s^{-1}$ | $3.78 \pm 0.06$ | $3.77 \pm 0.07$ | 0.79 | $3.77 \pm 0.07$ | 0.76 | $3.73 \pm 0.07$ | 2.82 * |
| | 225 | $3.98 \pm 0.07$ | $3.99 \pm 0.07$ | 0.47 | $3.97 \pm 0.08$ | 0.34 | $3.94 \pm 0.08$ | 2.15 |
| (cPs) | 90 | $4.34 \pm 0.10$ | $4.36 \pm 0.11$ | 0.63 | $4.30 \pm 0.10$ | 1.15 | $4.26 \pm 0.10$ | 2.95 * |
| | 45 | $4.71 \pm 0.13$ | $4.72 \pm 0.15$ | 0.28 | $4.65 \pm 0.14$ | 1.17 | $4.58 \pm 0.14$ | 2.20 |

$* = p < 0.05$ $** = p < 0.01$

## In-vivo Untersuchung zur antiischämischen Wirkung

### Versuchsdurchführung

Klinische Untersuchungen, die besonders von ERHLY[1] ausgeführt wurden, zeigen, daß bei Patienten mit peripherer arterieller Verschlußkrankheit der Gewebe-Sauerstoff-Partialdruck als Ausdruck der nutritiven Durchblutung im ischämischen Muskel, verglichen mit einer gesunden Extremität, herabgesetzt ist. Für die Untersuchung von Substanzen auf antiischämische Wirkung ist es deshalb sehr vorteilhaft, ein Tiermodell zu verwenden, bei dem analog zur klinischen Situation eine Ischämie durch einen herabgesetzten Sauerstoff-Partialdruck signalisiert wird. Ein solches Tiermodell kann man durch eine Ligatur an der A. femoralis der Ratte erhalten. Aufgrund von Untersuchungen von Substanzen, von denen bekannt ist, daß sie klinisch wirksam sind (Verlängerung der schmerzfreien Gehstrecke), ist bekannt, daß die Verbeserung der Durchblutung in der Mikrozirkulation bei diesem Tiermodell ebenso wie in der Klinik durch einen Anstieg des Gewebe-Sauerstoff-Partialdruckes angezeigt wird. Daraus läßt sich ableiten, daß das Tiermodell für Substanzuntersuchungen als sehr geeignet einzustufen ist. Es wurde daher verwendet, um herauszufinden, ob Doxaminol (BM 10.188) im ischämischen Gewebe eine Verbesserung der Geweberperfusion bewirkt.

4

## Methode

Bei 6 Ratten (Gewicht 450—550 g) wurde in kurzzeitiger Penthrane-Narkose auf der rechten Seite die A. femoralis zweifach ligiert. Anschließend wurde der Hautschnitt wieder vernäht. Das gleiche geschah an 5 weiteren Ratten, die als Kontrolltiere dienten. Alle Tiere erholten sich problemlos von diesem Eingriff. Ab dem nächsten Morgen erhielten die Ratten der Substanzgruppe 2 × täglich 4 mg/kg BM 10.188 p.o. (Chargen-Nr. 809002 A), in 5 ml/kg 1% Methylzellulose suspendiert, über einen Zeitraum von 5 Tagen. Bei den Kontrolltieren wurde das gleiche Volumen, jedoch ohne Substanz, appliziert. An dem auf die letzte Substanzgabe folgenden Tag wurden jeweils alle Tiere einer Gruppe der Sauerstoff-Partialdruckmessung unterzogen. Dabei erhielten die Ratten zur Narkose je nach Größe 1,3-2 ml 5% Thiobutabarbital (Inaktin®). Dann wurde auf beiden Seiten der M. gastrocnemicus freigelegt und die Fascie sehr vorsichtig entfernt. Anschließend wurde auf den Muskel die Mehrdrahtoberflächenelektrode nach KESSLER, LÜBBERS[2,3] (Bezugsquelle Fa. Eschweiler, Kiel) aufgesetzt.

Die Sauerstoffpartialdruckkurven, die von jedem einzelnen der 8 Drähte einer Elektrode registriert werden, kommen auf dem Monitor eines Computers zur Darstellung (Hard- und Software: Fa. Bruins Instruments, München). Für die Auswertung der dabei anfallenden Daten wurde die sogenannte Integralmethode verwendet. Darunter versteht man die Mittelung des Integrals unter jeder einzelnen der 8 Sauerstoffpartialdruckkurven über einen bestimmten, frei wählbaren Zeitraum. In unseren Versuchen betrug dieser 30 Minuten. Neben dem Gewebe-Sauerstoff-Partialdruck wurde außerdem der Blutdruck (Dehnungsmeßstreifenaufnehmer von der Fa. Bell und Howell, Pasadena/Kalifornien) und die Herzfrequenz (Pulsfrequenzmeßgerät PFM 2 Mino, Fa. Michael Nowak, Berlin) mittels eines Katheters in der Carotis gemessen. Am Anfang und am Ende eines jeden Versuches wurde außerdem der arterielle $pO_2$, pH und $pCO_2$ bestimmt. Um die Konstanz des Sauerstoffangebotes zu gewährleisten, wurden die Tiere beatmet (Atemfrequenz = 55/min. $P_{exspiratorisch}\, O_2$ = 18 Vol.% und $P_{exspiratorisch}\, CO_2$ etwa 5 Vol.%, registriert vom Atemgasanalysator Engström Eliza Duo, Schweden). Blutdurck, Herzfrequenz und $P_{exsp.}\, CO_2$ wurden auf einem 4-Kanal-Schreiber der Fa. Schwarzer, München (Physiopolygraph Varioscript), kontinuierlich registriert. Pro Tier dauerte ein solches Experiment etwa 1 Stunde.

## Resultate

Aus Tab. 1 geht hervor, daß sich in der Kontrollgruppe die mittleren Gewebe-Sauerstoff-Partialdrucke in der linken und der rechten Wade signifikant unterscheiden. Man erkennt außerdem, daß unter dem Einfluß von BM 10.188 die mittleren $pO_2$-Werte auf der ligierten Seite um etwa 4 mm Hg zunehmen, während am anderen, nicht ligierten Bein, eine Abnahme von etwa 12 mm Hg zu verzeichnen ist. Die führt dazu, daß der signifikante Links-Rechts-Unterschied so deutlich verringert wird, daß statistisch im Rahmen der Streuung ken Unterschied mehr nachweisbar ist.

Welche hämodynamischen Veränderungen die Gabe von BM 10.188 bewirkt hat, ist in Tab. 2 dargestellt. Demnach tritt kein statistisch signifikanter Effekt auf. Man erkennt jedoch, daß vor allem der systolische Blutdruck deutlich über dem Kontrollwert liegt. Weder die exspiratorischen Volumenanteile an $O_2$ und $CO_2$ noch pH, $pO_2$ und $pCO_2$ im arteriellen Blut zeigen auffällige Veränderungen. Alle genannten Parameter bewegen sich im Normbereich.

LITERATUR

(1) EHRLY, A.
Verbesserung der nutritiven Durchblutung bei peripheren ischämischen Erkrankungen.
VASA Suppl. *11*, (1983)

(2) KESSLER, M.:
Possibilities of measuring oxygen pressure fields in tissue by multiwire platinum electrodes.
Progr. resp. Res. *3*, 136—147 (1969)

(3) LÜBBERS, D. W.:
Principle of construction and application of various platinum electrodes.
Progr. Resp. Res. *3*, 136 (1969)

TABELLE 1

Die Ergebnisse der Gewebesauerstoffpartial-Druckmessung im Rattenmodell mit der chronischen Ligatur.
Doxaminol (BM 10.188): 4 mg/kg p.o. in Methylzelluloselösung suspendiert (6 Ratten)
Kontrollgruppe: Gleiches Volumen an Methylzelluloselösung (5 Ratten)

| Tier Nr. | BM 10.188 | | Kontrollgruppe | |
|---|---|---|---|---|
| | nicht ligiert | ligiert | nicht ligiert | ligiert |
| 1 | 25,3 | 21.3 | 34.0 | 9,1 |
| 2 | 37,1 | 18,6 | 41,8 | 29,9 |
| 3 | 18,4 | 10,9 | 46,1 | 26,0 |
| 4 | 29,2 | 35,5 | 40,4 | 7,0 |
| 5 | 30,9 | 32,9 | 39,3 | 22,7 |
| 6 | 27,7 | 20,6 | — | — |
| $\bar{x} \pm s_{\bar{x}}$ | 28,1 ± 2,5 | 23,0 ± 3,6 | 40,3 ± 2,0 | 18,9 ± 4,6 |

ns.      p < 0,01

t-Test für gepaarte Daten

TABELLE 2

Die hämodynamischen Meßergebnisse

Substanzgruppe: Doxaminol (BM 10.188) p.o., suspendiert in Methylzelluloselösung (6 Ratten)
Kontrollgruppe: Gleiches Volumen an Methylzelluloselösung (5 Ratten)

Ein statistischer Vergleich wurde mit dem zweiseitigen WILCOXON-U-Test (p ≤ 0,05) durchgeführt. Es trat keine Signifikanz auf.

| Ratte Nr. | BMN 10.188 | | | Kontrolle | | |
|---|---|---|---|---|---|---|
| | Herzfrequenz (Schläge/min) | $p_{syst.}$ (mm Hg) | $P_{diast.}$ (mm Hg) | Herzfrequenz (Schläge/min) | $p_{syst.}$ (mm Hg) | $P_{diast.}$ (mm Hg) |
| 1 | 370 | 165 | 135 | 430 | 140 | 110 |
| 2 | 320 | 170 | 135 | 380 | 140 | 115 |
| 3 | 340 | 160 | 125 | 425 | 180 | 145 |
| 4 | 170 | 195 | 150 | 325 | 125 | 95 |
| 5 | 330 | 165 | 130 | 360 | 145 | 120 |
| 6 | 260 | 160 | 125 | — | — | — |
| $\bar{x} \pm s_x$ | 298 ± 73 | 169 ± 13 | 133 ± 9 | 384 ± 44 | 146 ± 20 | 117 ± 18 |

Gegenstand der Erfindung ist nun die neue Verwendung von Doxaminol oder seinen physiologisch verträglichen Salzen zur Herstellung von Arzneimitteln zur Behandlung der peripheren arteriellen verschlußkrankheit.

Physiologisch verträgliche Salze lassen sich aus der freien Base mit Hilfe anorganischer oder organischer Säuren in bekannter Weise herstellen. Doxaminol und ihre Salze können in flüssiger oder fester Form enteral und parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält. Derartige Zusätze sind z.B. Tartrat- und Citratpuffer, Äthanol, Komplexbildner (wie Äthylendiamin-tetraessigsäure und deren nichttoxische Salze), hochmolekulare Polymere (wie flüssiges Polyäthylenoxid) zur Viskositätsregulierung.

Feste Trägerstoffe sind z.B. Stärke, Lactose, Mannit, Methylcellulose, Talkum, hochsidperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykole); für orale Applikation geeignete Zubereitungen können gewünschtenfalls Geschmacks- und Süßstoffe enthalten.

Die Dosierung kann von verschiedenen Faktoren abhängen, wie Applikationsweise, Spezies, Alter und/ oder individuellen Zuständen. Bei Menschen von ca. 70 kg Körpergewicht ist normalerweise von einer Dosis von 25—500 mg/Tag, vorzugsweise 50—200 mg/Tag, in 1—4 Einzeldosen auszugehen.

Arzneimittelformulierungen enthalten daher vorzugsweise 50—100 mg Wirkstoff.

## Patentanspruch

Verwendung von N - Methyl - N - [2 - (6,11) - dihydrodibenz[b,e]oxepin - 11 - yl)ethyl] - 1 - amino - 3 - phenoxy - propanol - (2) sowie deren physiologisch verträgliche Salze zur Herstellung von Arzneimitteln zur Behandlung der peripheren arteriellen Verschlußkrankheit.

## Revendication

Utilisation de N - méthyl - N - [2 - (6,11) - dihydrodibenz[b,e]oxépine - 11 - yl)ethyl] - 1 - amino - 3 - phéneoxy - propanol - (2) ainsi que leurs sels physiologiquement compatible pour l'obtention des medicaments destinés au traitement d'une maladie résultant de l'occlusion artérielle périphérique.

## Claim

Use of N - Methyl - N - [2 - (6,11) - dihydrodibenz[b,e]oxepin - 11 - yl)ethyl] - 1 - amino - 3 - phenoxy - propanol - (2) as well as their physiologically acceptable salts for the manufacture of medicaments for the treatment of peripheral artery occlusions.